# EUROPEAN PATENT SPECIFICATION

(11) **EP 1 474 080 B1**
(45) Date of publication and mention of the grant of the patent: **09.05.2007**
(21) Application number: 03704330.4
(22) Date of filing: 08.02.2003
(51) Int. Cl.: A61F 2/44

(54) **IMPLANT FOR SPINE**
WIRBELSÄULENIMPLANTAT
IMPLANT RACHIDIEN

(30) Priority: 13.02.2002 DK 200200217
(43) Date of publication of application: 10.11.2004
(73) Proprietor: Danfoss A/S, 6430 Nordborg (DK)
(72) Inventor: CHRISTENSEN, John, DK-6430 Nordborg (DK); PEDERSEN, Hans, Jørgen, 71720 Oberstenfeld (DE)
(86) International application number: PCT/DK2003/000081
(87) International publication number: WO 2003/068111

(56) References cited:
- EP-A- 0 560 279
- EP-A- 1 088 533
- WO-A-01/15637
- US-A- 5 782 919

## Description

The invention concerns an implant for replacing an element in a vertebral or cervical spine, which implant comprises a base body manufactured of a substantially X-ray penetrable material.

The stability and the function of the spine are based on the relations between spinal bodies (corpora), discs (disci intervertebrales), ligaments and musculature. Between any two spinal bodies of the spine is arranged a disc, which comprises a soft cartilaginous disc surrounded and enclosed by a fibrous ring (annulus fibrosus), which prevents the cartilaginous disc from leaving the space between the spinal bodies.

The disc can be displaced (slipped disc) or damaged, for example because of sedentary work, overloading, traffic accidents or illness, and problems with back or neck have become an everyday occurrence for many people. A slipped disc, meaning that the rear part of the annulus fibrosus has been hollowed or burst, can cause the disc to press against the spinal cord or a nerve root, which causes heavy pain or maybe paresis. In other cases, the disc has been overloaded or aged in such a way that the disc gets hard and dehydrated. In both cases, the disc shrinks and does not completely fill the space between the spinal bodies. This involves the risk of instability of the spine, reduced mobility and pain. In more severe cases it is necessary to remove one or more of these discs in an operation and replace them by implants.

Correspondingly, a spinal body can be damaged, for example by lateral displacement, torsion or axial compression, caused by a fall, a header in too low water or a traffic accident. Finally, spinal bodies can be damaged by illness, for example cancer. Common for these cases is that it may be necessary to remove a damaged spinal body and replace it by an implant.

During an operation, where one or more elements are removed, muscular and connective tissue is intersected, and it is necessary to make sure that the implant is fixed to neighbouring spinal elements as fast as possible, to ensure stability of the spine. For example, this fixation occurs in that bone from the two neighbouring spinal bodies grows onto the implant. It is important to be able to establish, whether or not a suitable ingrowth of bone onto the implant has taken place, and thus, whether the stability of the spine is ensured. With a traditional implant, which is made of a metal, it may be difficult to establish, whether an ingrowth has taken place, as metal is X-ray proof, which means that it creates shadows in the X-ray picture. Therefore, traditional implants made of metal are not optimal in connection with implants for the spine, as it is difficult or even impossible to determine, whether or not an ingrowth has taken place. An attempt to remedy this appears from EP 1 088 533 Al, which describes an implant as mentioned above, which consists of a basket-like container, which is made of interwoven threads or threads of metal, openings being formed between the threads, which permit X-rays to penetrate. However, this implant is very expensive in production, and besides, it is difficult to obtain a sufficient compression strength with this basket-like implant.

As appears from US patent No. 5,425,772, an implant made of, for example, polymer, maybe reinforced with fibres, can be used instead, as such an implant is X-ray penetrable, and therefore creates no shadows in an X-ray picture. However, this implant has the disadvantage that it cannot be seen from an X-ray picture, if the implant is correctly placed.

Besides, from the US patent No. 6,146,422 is known a disc implant, which is made of an X-ray penetrable material, such as a polymer, with two punctiform metal markers, which can be seen on an X-ray picture. With this embodiment, it is to some degree possible to determine the placing of the implant. It is difficult, however, to establish whether the bone grows close to the implant.

A document WO 01/15637 describes and relates to an intervertebral implant with a hollow, cylindrical shape and having a covering surface, a base surface, a hollow, cylindrical wall with one outer surface area, and one inner surface area), in addition to one central hollow, cylindrical shaft (6). The implant is composed of at least 95 % by volume of an X-ray-transparent material and highly biocompatible material, such as titanium or tantalum.

It is a purpose of the present invention to provide an implant, which enables both determination of the placing of the implant and of the bone growth around and onto the implant.

With this purpose, the implant according to the invention is defined in claim 1 and includes a surface modification comprising an X-ray proof material, which ensures that an X-ray will show, whether the implant is correctly placed. As the bone can be seen clearly on an X-ray picture, whether the bone grows around the implant and onto the surface, which can now be seen on an X-ray picture.

Of course, the simplest method is to modify the surface of the whole implant, but it is preferred that the surface modification merely comprises selected areas of the implant, preferable only surfaces facing each other, so that both areas with surface modification and areas without surface modification exist. The areas without surface modification form some kind of "windows", which are X-ray penetrable and leave no shadows on an X-ray picture. This makes it easier to see, whether the modified surface areas are placed as desired and whether a growth of the bone has taken place around and onto the implant.

The surface modification can be any X-ray proof material, but according to an embodiment the surface modification is a refractory metal, which is X-ray proof and generally very resistant to corrosion.

It is particularly preferred that the surface modification is made of tantalum, which is X-ray proof and has appropriate, biocompatible properties, which ensure that the implant is accepted by the body and that bone can grow onto the implant itself.

In an embodiment, the surface modification is placed on at least a share of the top side and/or the bottom side of the base body, so that these surfaces appear clearly on the X-ray picture, and it can be determined, whether the implant is placed correctly and close to the neighbouring spinal bodies.

In an embodiment, the base body encloses, completely or partly, a through hollow, which extends from the top side to the bottom side of the base body. This through hollow is well suited for ingrowth of bone, thus ensuring to a higher extent a fixing of the implant in the spine, which improves the stability of the spine.

It may be advantageous that the surface modification is placed on at least a share of the hollow sidewall, as thus it can be established, if the bone grows close around the implant through the hollow and maybe grows onto the surface.

According to the invention, the implant is characterised in that it comprises at least one net, suspended across the hollow of the base body. This creates some kind of shelf, on which a bone-growth supporting material can be placed. A further advantage of this net is that ingrowth into the net will prevent a relative displacement of the implant in relation to the neighbouring spinal bodies.

In many cases, one single net will be sufficient and suitable, but in some cases it may be appropriate that the implant comprises two nets, suspended at a certain distance from each other across the hollow, substantially in parallel with the top side and/or the bottom side of the base body. With this implant, a pocket has been created, in which bone-growth material can be placed, meaning that the bone-growth material is better secured against falling off, and at the same time it improves the chances of a fast ingrowth of bone into a net, so that an early protection against displacement of the implant is achieved.

Further, it may be appropriate that the surface modification is placed on the net, so that it is covered with an X-ray proof material, or that the net is made of threads of an X-ray proof material, like tantalum or platinum, as this will make the net appear clearly on an X-ray picture, which makes it easy to see, if an ingrowth of bone into the net has taken place.

One embodiment is characterised in that the base body has substantially the shape of a horseshoe with two branches. This embodiment has turned out to be particularly appropriate for an implant meant for replacing an element in the spine.

An implant, whose base body has the shape of a closed ring with a circular, oval or polygonal basic shape has proved to be well suited for insertion in the cervical spine, among other things because here the dimensions of the elements are relatively small.

Fixation in the period, until ingrowth has taken place, can, among other things, be achieved by a so-called Harrington-system, which consists of long bars inserted around the spine with the purpose of fixing it, but such systems are inconvenient for the patient, and often there is a risk that the bars work loose. In order to achieve a better fixation, the implant is, in one embodiment, provided with diagonal, through holes from an outside of the base body to the top side and/or the bottom side of the base body, respectively, meaning that the implant can be fixed by means of screws or brads to one or both of the neighbouring spinal bodies. This is particularly important during the period, where ingrowth of bone into the implant has not yet taken place.

The invention also concerns a method for manufacturing an implant for replacement of a disc in a spine.

In the following, the invention will be described in detail on the basis of an embodiment example and with reference to the enclosed drawings, showing:
- Fig. 1: an implant having the shape of a horseshoe,
- Fig. 2: a cross-section II-II in Fig. 1
- Fig. 3: an implant having the shape of a ring
- Fig. 4: a cross-section in Fig. 3
- Fig. 5: a section through an implant provided with spikes,
- Fig. 6: an implant according to the invention provided with a net,
- Fig. 7: a section VII-VII in Fig. 6
- Fig. 8: a ring-shaped implant according to the invention provided with a net
- Fig. 9: a cross-section in Fig. 8

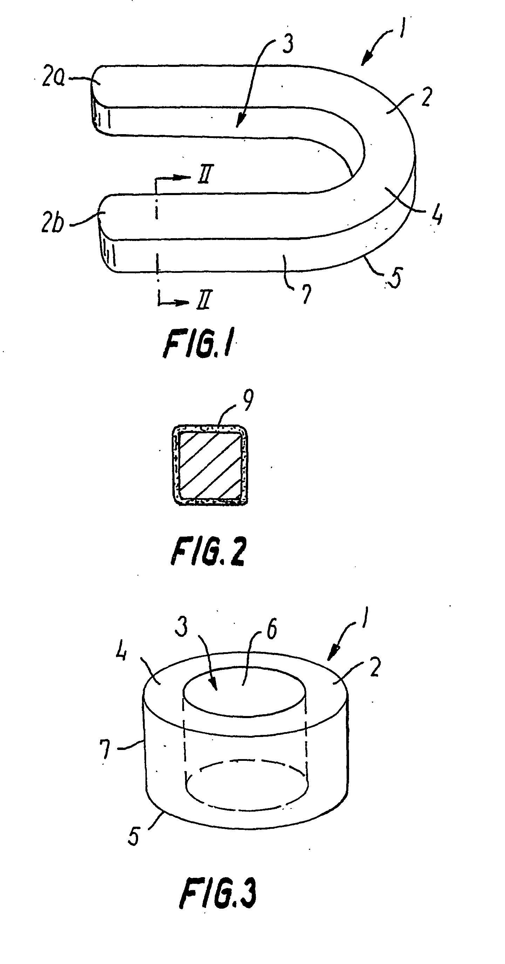

From Fig. 1, which schematically shows an implant 1, it appears that the implant 1 comprises a horse-shoe shaped base body 2 with two branches 2a, 2b, which partly enclose a through hollow 3, extending from a top side 4 of the base body 2 to a bottom side 5 of the base body 2.

After the implantation of an implant it is essential to be able to determine, whether the implant is placed correctly, and whether an ingrowth of bone into and towards the implant takes place to ensure that the implant is safely fixed on the neighbouring bones. It is therefore preferred that the implant is visible on an X-ray picture, but at the same time it is particularly preferred that the implant does not create shadows on the X-ray picture, which will make it difficult or impossible to determine, whether or not an ingrowth into the hollow 3 of the implant has taken place. As appears from Fig. 2, the implant 1 can have a complete or partial surface modification 9, which is completely or partly X-ray proof. Particularly in connection with a base body 2, which is made of an X-ray penetrable material, this has the advantage that the surface of the implant is clearly visible on an X-ray picture, and it is therefore easy to determine, whether a bone ingrowth into the implant has taken place. The surface modification can be any X-ray proof material suited for implantation into a living organism, meaning that it is preferred that the material is biocompatible. Examples of suitable materials are refractory metals, such as niobium and tantalum, as well as the metal titanium. It can be mentioned that a surface modification 9 of tantalum with a thickness of 5 to 10 µm will provide the desired X-ray proofness, and at the same time there will be no disturbing shadows on the X-ray picture, as a surface modification with this thickness will still be X-ray penetrable to some extent.

An implant 1 of this type, comprising a base body 2 with horse-shoe shape has turned out to be particularly well suited for replacement of an element in a vertebral spine, whereas in connection with implants for replacement of elements in the cervical spine it is usually preferred that the implant 1 as shown in Fig. 3 comprises a base body 2 in the shape of a closed ring, which can, for example, be circular or rectangular, said base body 2 enclosing a through hollow 3.

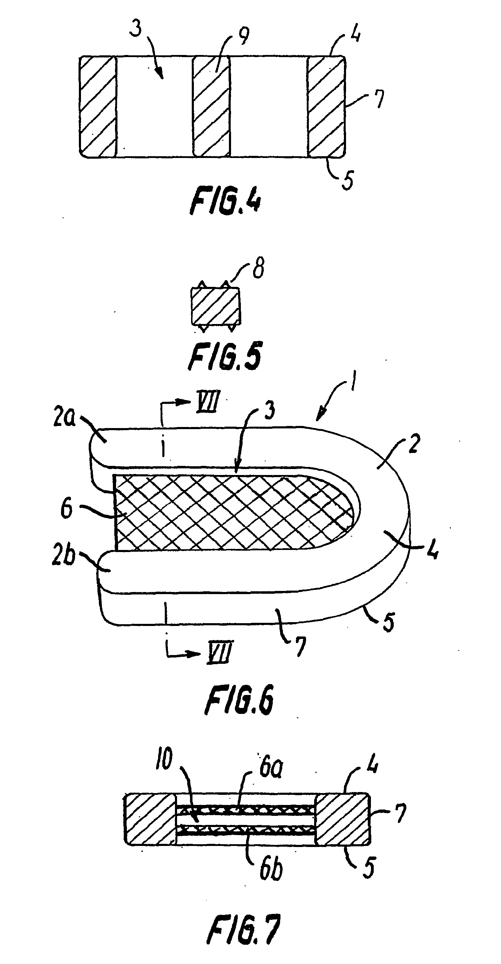

The surface modification can cover the whole implant or, as shown in Fig. 4, only a limited share, such as facing parts of side faces of the base body inside the hollow. Thus, it can be achieved that some kind of X-ray penetrable "windows" are formed in the areas having no surface modification, so that an X-ray picture makes it possible to determine, whether ingrowth of bone through the hollow of the implant and towards the surface of the implant has taken place, as the surface modified areas will be visible on the X-ray picture.

The base body 2 can have through holes (not shown) from a side face 7 to the top side 4 and/or the bottom side 5, respectively, of the base body 2. These holes permit the insertion of brads, screws or the like for fixing the implant in relation to the neighbouring spinal bodies.

Thus, it is obtained that the implant 1 cannot be displaced during the period, where an ingrowth of bone material through the implant has not yet taken place. However, it must be mentioned that in connection with implants for replacement of an element in the cervical spine it will often not be possible to fix the implant to the neighbouring spinal bodies by means of screws or brads, as the spinal bodies of the cervical spine are relatively small. Additionally to this, or instead of this, the top side and or the bottom side 4, 5 of the base body 2 can be rugged, or, as appears from Fig. 5, be provided with relatively pointed spikes or combs 8, which expediently extend in different directions. This minimises the risk that the implant is displaced in relation to the neighbouring spinal bodies.

At the top side 4, the bottom side 5, or somewhere between the top side 4 and the bottom side 5, a net 6 can be suspended, as shown in Figs. 6, 7, 8 and 9, which net 6 is substantially parallel to the top side 4 and/or the bottom side 5. This net can be provided with a layer of bone-growth supporting material, such as the product Colloss® from the German company Ossacur AG.

The document WO 01/15637 does not describe the use of a bone-growth supporting material, or the introduction of such a net (6).

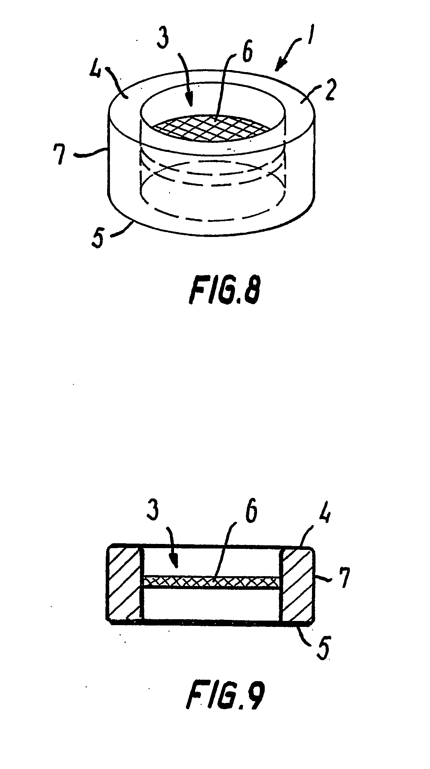

As shown in Figs. 8 and 9, the implant 1 can comprise one single net 6, or, as shown in Fig. 7, which is a section VII-VII in Fig. 6, the implant 1 can advantageously comprise two nets 6a, 6b, which are suspended between the two branches 2a, 2b of the base body 2. This will advantageously form a pocket 10, in which bone-growth supporting material can be placed.

The base body 2 of the implant 1 can be made of a fibre-reinforced polymer, in which the fibres are further used for creating the net 6. In this way, the net 6 can expediently be produced integrally with the base body 2. Alternatively, the net 6 can be mounted on the base body 2. Examples of suited polymers are PEEK (polyetheretherketone) and PEKEKK, and the fibres can, for example, be carbon fibres.

The implant can also be made of graphite, carbon fibre-reinforced graphite or metal. In this case, the base body can be made of metal in bulk, extruded, maybe with a net, or sintered powder or foamed material.

Suited dimensions of an implant for replacement of a disc in the vertebral spine of an adult will be a height of about 10 to 13 mm, a width of about 8 mm and an opening, whose largest dimension is about 25 mm. An implant for a child will of course be somewhat smaller.

Suited dimensions of an implant for replacement of a disc in the cervical spine of an adult will be a height of about 8 mm, a width of about 20 mm and a depth of about 10 mm. The hollow can, for example, have a cross section of about 4 times 12 mm.

In case that the implant must replace a spinal body, it will be obvious for a person skilled in the art that the implant must be substantially higher.

The figures show implants with parallel top and bottom sides, but in some cases it may be appropriate for the implant to be wedge-shaped, so that the heights of the front and the rear sides of the implant are different, which will give the desired curvature of the spine.

## Claims

1. Implant (1) for replacing an element in a vertebral or cervicial spine, which implant (1) comprises a base body (2) manufactured of a substantially X-ray penetrable material, said base body (2) comprising a through hollow (3), wherein the implant has a surface modification (9) comprising an X-ray proof material, **characterised in that** the implant (1) comprises at least one net (6), suspended across the hollow (3) of the base body (2) and adapted to contain a bone-support growth material.

2. Implant (1) according to claim 1, wherein the surface modification (9) merely comprises selected areas of the implant (1), preferable only surfaces opposite each other.

3. Implant (1) according to any of the preceding claims, wherein the surface modification (9) is a refractory metal.

4. Implant (1) according to claim 3, wherein the surface modification (9) is made of tantalum.

5. Implant (1) according to claim 3, wherein the surface modification (9) is placed on at least a share of the top side (4) and/or the bottom side (5) of the base body (2).

6. Implant (1) according to any of the preceding claims, wherein the base body (2) encloses, completely or partly, a through hollow (3), which extends from the top side (4) to the bottom side (5) of the base body (2).

7. Implant (1) according to claim 6, wherein the surface modification (9) is placed on at least a share of the hollow (3) sidewall.

8. Implant (1) according to claim 6 or 7, wherein the implant (1) comprises two nets (6a, 6b), suspended between two branches (2a, 2b) of the base body (2) each adapted to contain a bone-support growth material.

9. Implant (1) according to claim 8, wherein the surface modification (9) is placed on the net (6), or that the net (6) is made of threads of an X-ray proof material, like tantalum or platinum.

10. Implant (1) according to any of the preceding claims, wherein the base body (2) has the shape of a horseshoe with two branches (2a, 2b).

11. Implant (1) according to any of the preceding claims, wherein the base body (2) has the shape of a closed ring with a circular, oval or polygonal basic shape.

12. Implant (1) according to any of the preceding claims, wherein the implant (1) is provided with diagonal, through holes from an outside (7) of the base body (2) to the top side and/or the bottom side (4, 5) of the base body (2).

## Patentansprüche

1. Implantat (1) zum Ersetzen eines Elements in einer Rückenwirbelsäule oder Halswirbelsäule, wobei das Implantat (1) einen Grundkörper (2), hergestellt aus einem im wesentlichen von Röntgenstrahlen durchdringlichen Material, aufweist, und der Grundkörper (2) einen durchgehenden Hohlraum (3) aufweist, in dem das Implantat eine Oberflächenmodifizierung (9) aufweist, die ein Röntgenstrahl-beständiges Material enthält, **dadurch gekennzeichnet, dass** das Implantat (1) mindestens ein Netz (6) aufweist, das über den Hohlraum (3) des Grundkörpers (2) aufgehängt und zum Tragen eines Knochenwachstums-Förderungsmaterial geeignet ist.

2. Implantat (1) nach Anspruch 1, in dem die Oberflächenmodifizierung (9) lediglich ausgewählte Bereiche des Implantats (1) deckt, vorzugsweise nur gegenüberliegende Oberflächen.

3. Implantat (1) nach jedem der vorhergehenden Ansprüche, in dem die Oberflächenmodifizierung (9) ein feuerfestes Metall ist.

4. Implantat (1) nach Anspruch 3, in dem die Oberflächenmodifizierung (9) aus Tantal hergestellt ist.

5. Implantat (1) nach Anspruch 3, in dem die Oberflächenmodifizierung (9) zumindest auf einem Teil der Oberseite (4) und/oder der Bodenseite (5) des Grundkörpers (2) angebracht ist.

6. Implantat (1) nach jedem der vorhergehenden Ansprüche, in dem der Grundkörper (2) einen durchgehenden Hohlraum (3) ganz oder teilweise umgibt, der sich von der Oberseite (4) zur Unterseite (5) des Grundkörpers (2) erstreckt.

7. Implantat (1) nach Anspruch 6, in dem die Oberflächenmodifizierung (9) zumindest auf einem Teil der Seitenwand des Hohlraumes (3) angebracht ist.

8. Implantat (1) nach Anspruch 6 oder 7, in dem das Implantat (1) zwei Netze (6a, 6b) aufweist, die zwischen zwei Abzweigungen (2a, 2b) des Grundkörpers (2) aufgehängt sind und zum Tragen eines Knochenwachstums-Förderungsmaterial geeignet sind.

9. Implantat (1) nach Anspruch 8, in dem die Oberflächenmodifizierung (9) auf dem Netz (6) angebracht ist, oder das Netz (6) aus Fasern eines Röntgenstrahl-beständigen Materials, wie beispielsweise Tantal oder Platin, besteht.

10. Implantat (1) nach jedem der vorhergehenden Ansprüche, in dem der Grundkörper (2) die Form eines Hufeisens mit zwei Abzweigungen (2a, 2b) hat.

11. Implantat (1) nach jedem der vorhergehenden Ansprüche, in dem der Grundkörper (2) die Form eines geschlossenen Rings mit einer kreisrunden, ovalen oder polygonalen Grundform hat.

12. Implantat (1) nach jedem der vorhergehenden Ansprüche, in dem das Implantat (1) mit diagonalen, durchgehenden Löchern von einer Aussenseite (7) des Grundkörpers (2) zur Oberseite und/oder zur Unterseite (4, 5) des Grundkörpers (2) versehen ist.

## Revendications

1. Implant (1) destiné à remplacer un élément dans une colonne vertébrale ou cervicale, ledit implant (1) comprend un corps de base (2) fabriqué dans un matériau en grande partie perméable aux rayons X, ledit corps de base (2) comportant un creux traversant (3), dans lequel l'implant comprend une modification de surface (9) en un matériau imperméable aux rayons X, **caractérisé en ce que** l'implant (1) comprend au moins un filet (6) suspendu dans le creux (3) du corps de base (2) et adapté pour contenir un matériau qui favorise la croissance osseuse.

2. Implant (1) selon la revendication 1, dans lequel la modification de surface (9) comprend seulement des zones sélectionnées de l'implant (1), de préférence uniquement des surfaces en face les unes des autres.

3. Implant (1) selon l'une des revendications précédentes, dans lequel la modification de surface (9) est un métal réfractaire.

4. Implant (1) selon la revendication 3, dans lequel la modification de surface (9) est en tantale.

5. Implant (1) selon la revendication 3, dans lequel la modification de surface (9) est placée sur au moins une partie du côté supérieur (4) et/ou du côté inférieur (5) du corps de base (2).

6. Implant (1) selon l'une des revendications précédentes, dans lequel le corps de base (2) entoure en totalité ou en partie un creux traversant (3) qui s'étend depuis le côté supérieur (4) jusqu'au côté inférieur (5) du corps de base (2).

7. Implant (1) selon la revendication 6, dans lequel la modification de surface (9) est placée sur au moins une partie de la paroi latérale creuse (3).

8. Implant (1) selon la revendication 6 ou 7, dans lequel l'implant (1) comprend deux filets (6a, 6b) suspendus entre deux branches (2a, 2b) du corps de base (2), chacun des filets étant adapté à contenir un matériau qui favorise la croissance osseuse.

9. Implant (1) selon la revendication 8, dans lequel la modification de surface (9) est placée sur le filet (6) ou dans lequel le filet (6) est constitué de fils en un matériau imperméable aux rayons X comme le tantale ou le platine.

10. Implant (1) selon l'une des revendications précédentes, dans lequel le corps de base (2) est en forme de fer à cheval à deux branches (2a, 2b).

11. Implant (1) selon l'une des revendications précédentes, dans lequel le corps de base (2) est en forme d'anneau fermé dont la forme de base est circulaire, ovale ou polygonale.

12. Implant (1) selon l'une des revendications précédentes, dans lequel l'implant (1) comporte des trous traversant diagonaux depuis l'extérieur (7) du corps de base (2) jusqu'au côté supérieur et/ou au côté inférieur (4, 5) du corps de base (2).
